# EUROPÄISCHE PATENTSCHRIFT

(11) **EP 2 214 750 B1**
(45) Veröffentlichungstag und Bekanntmachung des Hinweises auf die Patenterteilung: **24.02.2016**
(21) Anmeldenummer: 08858108.7
(22) Anmeldetag: 02.12.2008
(51) Int. Cl.: A61M 1/00, A61F 13/02

(54) **WUNDABDECKUNGS-VERBINDUNGSVORRICHTUNG**
WOUND COVER CONNECTING DEVICE
DISPOSITIF DE LIAISON DE PANSEMENT

(30) Priorität: 07.12.2007 CH 19002007
(43) Veröffentlichungstag der Anmeldung: 11.08.2010
(73) Patentinhaber: Medela Holding AG, 6340 Baar (CH)
(72) Erfinder: LARSSON, Michael, 6300 Zug (CH)
(74) Vertreter: Clerc, Natalia
(86) Internationale Anmeldenummer: PCT/CH2008/000505
(87) Internationale Veröffentlichungsnummer: WO 2009/070905

(56) Entgegenhaltungen:
- US-A1- 2003 040 687
- US-A1- 2005 085 795
- US-A1- 2007 021 698

## Beschreibung

### Technisches Gebiet

Die Erfindung betrifft eine Wundabdeckungs-Verbindungsvorrichtung gemäss Oberbegriff des Patentanspruchs 1.

### Stand der Technik

Es ist bekannt, grosse oder schlecht heilende Wunden mit einer Unterdruck-Drainageeinheit zu behandeln. Die Wunde wird dabei mit einer Abdeckung, beispielsweise einer Folie oder einer steifen Haube, überdeckt, so dass ein Wundraum entsteht. In den Wundraum wird von aussen ein Drainageschlauch eingerührt, welcher mit einer Unterdruckpumpe verbunden ist, um Wundsekret aus der Wunde abzusaugen. Üblicherweise wird unter der Abdeckung eine Wundauflage auf die Wunde gelegt, um den Wundraum zu füllen und insbesondere um den Unterdruck gleichmässig über sie Wundoberfläche zu verteilen.

Vorzugsweise ist der Drainageschlauch an seinem wundseitigen Ende so gestaltet, dass der Unterdruck verteilt angelegt werden kann. Beispielsweise offenbart US 2005/0137539 ein längliches Schlauchende mit Perforationen.

JP 2006116162 zeigt ein vereinfachtes Wunddrainagesystem ohne Absorptionskissen in der Wunde, aber mit einer durchsichtigen Abdeckung, welche steif genug ist, um nicht den Boden des Wundbetts zu berühren. Der Drainageschlauch weist ein Ende auf, welches die Wundabdeckung durchsetzt, und in einem Endbereich mit mehreren Löchern versehen ist.

WO 2006/105892 beschreibt eine Verbindungsvorrichtung bzw. einen Saugkopf zur Verbindung von Schlauch und Abdeckung, wobei dieses Verbindungselement unterhalb der Abdeckung angeordnet ist und lediglich mit einem Anschlussstutzen aus dieser herausragt. Das Verbindungselement weist radial angeordnete Kanäle auf, durch welche abgesaugt werden kann.

In JP 2005334188 ist ein hohler Rahmen mit Perforationen vorhanden, welcher mit der Saugquelle verbunden ist.

WO 01/34223 schlägt vor, einen flachen, runden Saugkopf zu verwenden, welcher eine mittig angeordnete Saugöffnung und um diese Öffnung verteilt angeordnete, zum Wundbett hin gerichtete Erhöhungen aufweist.

Auch in US 2007/0032778 weist der Saugkopf in das Wundbett ragende Erhöhungen auf.

In den oben beschriebenen Beispielen befindet sich der Saugkopf jeweils unterhalb der Abdeckung im Wundbett. Dasselbe gilt für WO 2007/084792, US 2007/0129660, EP 1 772 160 und US 7 216 651.

In WO 03/018098 wird der Schlauch in ein Verbindungsstück eingeführt, welches auf der Oberseite der Abdeckung an dieser anhaftet.

Die bekannten Verbindungsarten weisen mehrere Nachteile auf. Das Erstellen der Verbindung ist meistens zeitaufwendig und verlangt eine gewisse Geschicklichkeit. Es besteht oft die Gefahr, dass die Verbindung nicht dicht ist. Des Weiteren können die Verbindungselemente bzw. Saugköpfe gemäss dem Stand der Technik entweder nicht gleichmässig über den Wundbereich verteilt absaugen oder sie sind relativ gross und benötigen innerhalb des Wundbetts viel Platz. Im letzteren Fall besteht die Gefahr, dass sie den Boden des Wundbetts kontaktieren und dem Patienten unnötig Schmerzen berei ten bzw. sich störend auf die Wundheilung auswirken.

US 2005/0085795 offenbart eine Verbindungsvorrichtung zur dichtenden Verbindung eines Drainageschlauchs mit einer Wundabdeckung. Diese Vorrichtung weist ein Rohr zur Aufnahme des Schlauchs und einen das Rohr umlaufenden Flansch auf, welcher auf der Wundabdeckung aufliegt.

### Darstellung der Erfindung

Es ist deshalb eine Aufgabe der Erfindung, eine Wundabdeckungs-Verbindungsvorrichtung zu schaffen, welche es ermöglicht, eine dichte Verbindung zwischen einem Schlauch und einer Wundabdeckung auf einfache Art und Weise zu erstellen und somit innerhalb kurzer Zeit eine Leitung zur Wunde zu legen.

Diese Aufgabe löst eine Wundabdeckungs-Verbindungsvorrichtung mit den Merkmalen des Patentanspruchs 1.

Die erfindungsgemässe Wundabdeckungs-Verbindungsvorrichtung zur dichtenden Verbindung eines Schlauchs und einer eine Wunde verschliessenden Wundabdeckung weist einen Grundkörper auf zur dichtenden Auflage auf einer Aussenseite der Abdeckung. Die Vorrichtung weist erfindungsgemäss mindestens ein Durchdringungselement auf zur Erzeugung einer Durchgangsöffnung in der Abdeckung.

Die erfindungsgemässe Wundabdeckungs-Vorrichtung ist somit ein Kopplungs- der Anschlussteil zur Verbindung von Wundabdeckung und Schlauch. Sie bildet somit ein Interface.

Der Schlauch kann, muss aber nicht ein Drainageschlauch zur Absaugung von Wundsekret sein. Es kann ebenso ein Schlauch sein, welcher ein Gas oder eine Flüssigkeit in das Wundbett zuführt. Die Verbindungsvorrichtung dient somit einerseits zum Verbinden mit einem Saugschlauch zur Beaufschlagung der Wunde mit Vakuum. Sie kann jedoch alternativ oder zusätzlich auch zur Zuführung von Medikamenten, Flüssigkeiten oder Gasen in die Wunde dienen.

In einer bevorzugten Ausführungsform ist das mindestens eine Durchdringungselement spitzig und/oder schneidend ausgebildet.

Das mindestens eine Durchdringungselement dient in einer Ausführungsform lediglich zum Erstellen eines Verbindungskanals zwischen Wundbett und Schlauch. Vorzugsweise ist das Durchdringungselement hohl und mit mindestens einer Durchgangsöffnung versehen ausgebildet, so dass die Absaugung bzw. Zuführung von Flüssigkeiten oder Gasen durch dieses Durchdringungselement erfolgen kann.

In einer bevorzugten Ausführungsform ist die Verbindungsvorrichtung als separates Teil ausgebildet, welches mit einem Drainageschlauch verbindbar ist. Insbesondere lässt sich der Schlauch auf dieses Teil aufschieben. In einer anderen, ebenfalls bevorzugten Ausführungsform ist die Verbindungsvorrichtung ein integraler Bestandteil des Diainageschlauchs.

Dank der erfindungsgemässen Vorrichtung kann die Wunde in einem ersten Schritt abgedeckt werden. Das Legen der Leitung in die Wunde, d.h. das Verbinden der Wundabdeckung mit dem Schlauch kann in einem zweiten und separaten Schritt erfolgen.

Zum Legen der Leitung muss lediglich die erfindungsgemässe Vorrichtung auf die Abdeckung aufgelegt werden und das mindestens eine Durchdringungselement durch die Abdeckung gepresst werden. In einer bevorzugten Ausführungsform erfolgt dieses Durchstossen alleine dadurch, dass die Vorrichtung mit genügendem Druck auf die Abdeckung aufgelegt wird. Sind die Durchdringungselemente stumpf ausgebildet, so muss der Druck höher sein, als wenn sie spitzig bzw. schneidend ausgebildet sind.

Da dabei keine Teile nachträglich unter die Abdeckung eingeführt werden müssen, ist die Gefahr, dass die Wände der Wunde berührt oder gestört werden, relativ gering. Das Legen der Leitung erfolgt für den Patienten somit wesentlich schmerzfreier als bei den bekannten Vorrichtungen. Des Weiteren kann dadurch auch die Wunde im Operationssaal mit der Abdeckung zugedeckt werden, der Patient transportiert und die Leitung erst auf der Pflegestation gelegt werden. Auch dies ist für den Patienten schmerzfreier, da einerseits das Personal auf der Pflegestation mehr Zeit aufwenden kann und der Patient zudem nicht mit einem bereits angeschlossenen Schlauch transportiert werden muss. Vorteilhaft ist zudem, dass die Vorrichtung mit den unterschiedlichsten Typen von Wundverschlüssen anwendbar ist. Die Vorrichtung ist ferner kostengünstig herstellbar.

Es ist eine weitere Aufgabe der Erfindung, eine Wundabdeckungs-Verbindungsvorrichtung zu schaffen, welche eine gleichzeitige Beaufschlagung eines möglichst grossen Wundbereichs ermöglicht.

Diese Aufgabe löst eine Wundabdeckungs-Verbindungsvorrichtung mit den Merkmalen des Patentanspruchs 2.

Die erfindungsgemässe Vorrichtung weist hierfür mehrere beanstandet zueinander angeordnete Durchdringungselemente auf, welche zur Durchstossung der Abdeckung an mehreren beabstandet zueinander angeordneten Stellen ausgebildet sind.

Vorzugsweise sind diese Durchdringungselemente gleichmässig über die gesamte Grundfläche des Grundkörpers angeordnet.

Weitere vorteilhafte Ausführungsformen gehen aus den abhängigen Patentansprüchen hervor.

### Kurze Beschreibung der Zeichnungen

Im Folgenden wird der Erfindungsgegenstand anhand von bevorzugten Ausführungsbeispielen, welche in den beiliegenden Zeichnungen dargestellt sind, erläutert. Es zeigen:
- Figur 1: einen Längsschnitt durch eine schematische Darstellung einer Wundabdeckungs-Verbindungsvorrichtung gemäss einer ersten Ausführungsform der Erfindung in Gebrauch;
- Figur 2: eine perspektivische Darstellung der Vorrichtung gemäss der ersten Ausführungsform;
- Figur 3: einen Längsschnitt durch die Vorrichtung gemäss Figur 2;
- Figur 4: eine Ansicht der Vorrichtung gemäss Figur 2 von oben;
- Figur 5: eine Ansicht der Vorrichtung gemäss Figur 2 von unten;
- Figur 6: eine Seitenansicht der Vorrichtung gemäss Figur 2 und
- Figur 7: einen Längsschnitt durch eine schematische Darstellung einer Wundabdeckungs-Verbindungsvorrichtung gemäss einer zweiten Ausführungsform der Erfindung in Gebrauch.

### Wege zur Ausführung der Erfindung

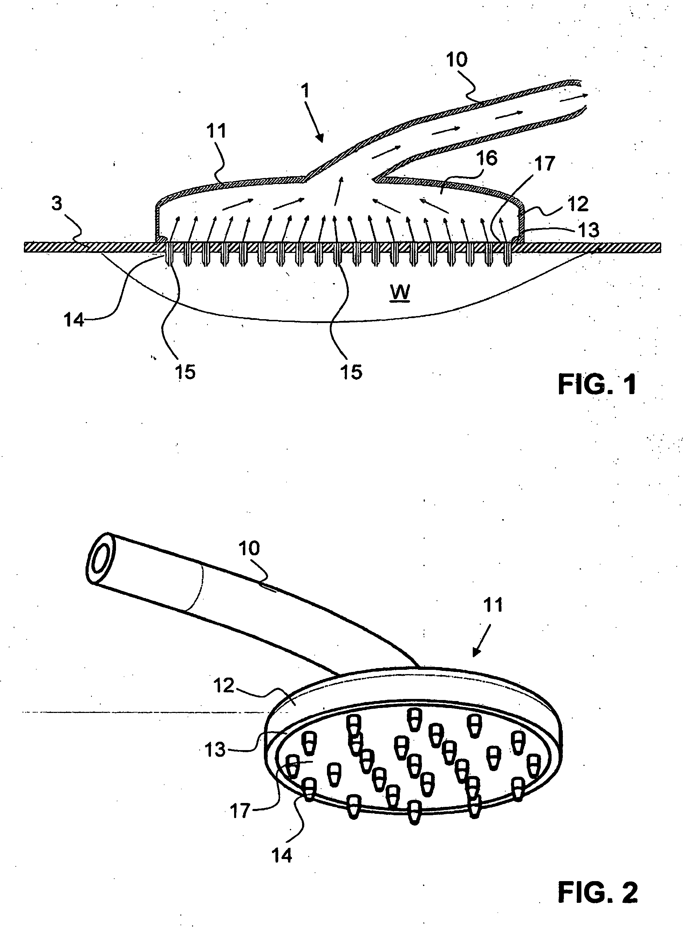

In Figur 1 ist ein Längsschnitt durch eine Wunde mit einem Wundbett W dargestellt, welches mit einer Wundabdeckung 3 luftdicht abgedeckt ist. Die Wundabdeckung 3 ist dabei vorzugsweise auf der die Wunde umgebenden, gesunden Haut befestigt, insbesondere mit Klebeband. Als Wundabdeckung 3 eignen sich die bekannten Wundabdeckungen, insbesondere Folien oder steife Kappen. Das Wundbett W kann natur belassen oder mit einer Wundauflage, insbesondere einem Schaumstoff oder einem anderen Wundfüllstoff bekannter Art, gefüllt sein.

Zur Förderung der Heilung wird die Wunde mit Unterdruck behandelt. Hierfür wird von einer Saugquelle, insbesondere einer Vakuumpumpe, eine Saugleitung durch die Abdeckung 3 bis in den Wundraum gelegt. Im Gegensatz zum Stand der Technik wird hierfür jedoch nicht ein Endstück eines Saugschlauchs durch die Wundabdeckung 3 hindurch in den Wundraum eingeführt, sondern es muss lediglich die erfindungsgemäss Verbindungsvorrichtung 1 auf der Wundabdeckung 3 aufgelegt und an diese angepresst werden.

Diese Verbindungsvorrichtung 1 weist einen Grundkörper 11 auf, an welchem auf einer Seite ein hohler Anschlussstutzen 10 angeformt ist. Der Grundkörper 11 weist an einer anderen, vorzugsweise an der gegenüberliegenden Seite eine Auflagewand 17 auf, welche mit Durchdringungselementen 14 versehen ist.

Der Grundkörper 11 weist eine Grundfläche auf, welche vorzugsweise um ein Vielfaches grösser ist als die Querschnittsfläche des Anschlussstutzens 10. Der Grundkörper 11 ist vorzugsweise starr bzw. formstabil ausgebildet. Insbesondere ist er aus Metall oder Kunststoff gefertigt. Vorzugsweise ist er gemeinsam einstückig mit dem Anschlussstutzen 10 ausgebildet.

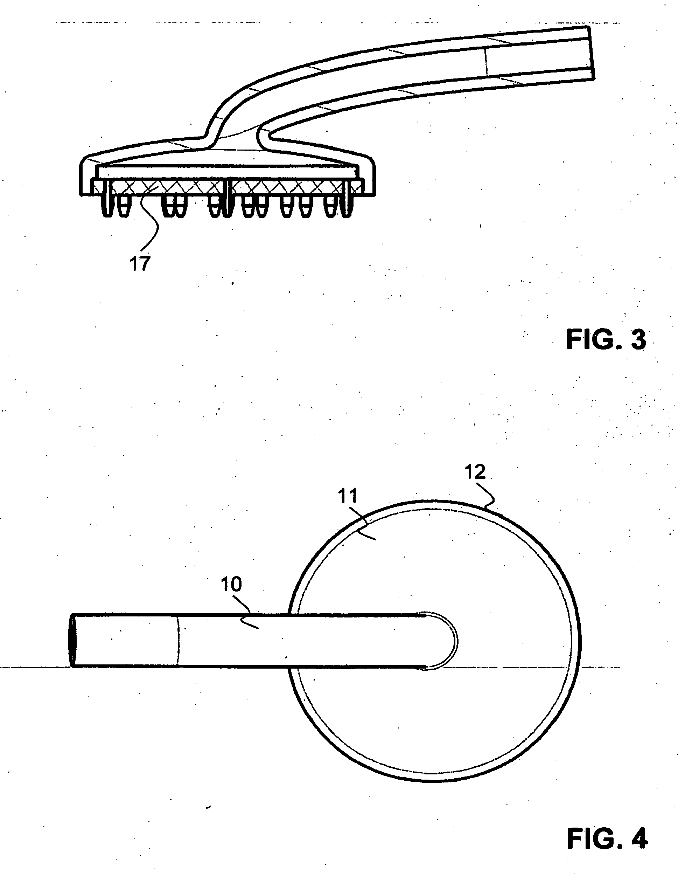

Der Grundkörper 11 ist im Querschnitt bzw. in der Draufsicht vorzugsweise rund ausgebildet, wie dies beispielsweise in Figur 4 erkennbar ist. Er kann jedoch auch eine rechteckige, ovale, sternförmige oder eine andere Grundform aufweisen. Vorzugsweise ist er relativ flach ausgebildet. Seine äussere Form kann beispielsweise einem Kopf einer Duschbrause entsprechen, so dass sein Längsschnitt annähernd eine rechteckige Form aufweist. Der Grundkörper 11 kann jedoch auch annähernd halbkugelförmig oder kegelstumpf- oder pyramidenförmig ausgebildet sein, so dass sein Längsschnitt annähernd die Form eines Halbkreises bzw. eines Dreiecks aufweist.

Der Grundkörper 11 ist nach unten, d.h. wundseitig offen ausgebildet und mit der Auflagewand 17 verschlossen. Dadurch wird im Innern des Grundkörpers 11 mindestens ein Hohlraum 16 gebildet. Es können auch mehrere, miteinander kommunizierende oder voneinander getrennte Hohlräume bzw. Durchgangskanäle vorhanden sein. Der mindestens eine Hohlraum 16 ist mit dem Kanal des Anschlussstutzens 10 verbunden. Dies ist in den Figuren 1 und 3 erkennbar.

Der Anschlussstutzen 10 ist vorzugsweise aus demselben Material gefertigt wie der Grundkörper 11, insbesondere aus Metall oder Kunststoff. Er kann seitlich am Grundkörper 11, d.h. am Mantel 12 des Grundkörpers 11, angeordnet sein oder an die obere, dem Wundbett abgewandte Seite des Grundkörpers 11 anschliessen. Im letzteren Fall kann er zudem senkrecht von dieser Seite abstehen oder, wie in den Figuren dargestellt, in einem nicht rechten Winkel dazu verlaufen.

Der Anschlussstutzen 10 kann als kurzes Rohrstück ausgebildet sein, auf welches ein Drainageschlauch aufgesteckt oder eingesteckt werden kann. Der Anschlussstutzen 10 kann jedoch auch gemeinsam mit dem Drainageschlauch einstückig ausgebildet sein. Im letzteren Fall bildet die erfindungsgemässe Verbindungsvorrichtung das wundseitige Ende des Drainageschlauchs. Der Drainageschlauch ist vorzugsweise aus Silikon gefertigt. Sein Ende kann jedoch aus einem anderen Material hergestellt sein.

Der Grundkörper 11 ist wundseitig mit einem Dichtungselement, insbesondere einem den Mantel 12 umlaufenden Dichtring 13 oder einer Dichtfläche versehen. Mit dem Mantel 12 liegt der Grundkörper 11 auf der Wundabdeckung 3 auf, wobei der Dichtring 13 eine luftdichte Verbindung bildet. Anstelle oder zusätzlich zum Dichtring 13 lässt sich der Grundkörper 11 auch mit bekannten Mitteln gegenüber der Abdeckung 3 dichten, beispielsweise kann er mit einem Klebeband angeklebt werden.

Der Dichtring 13 kann, wie in Figur 1 dargestellt, auf der Innenseite des Grundkörpers 11 bzw. des Mantels 12 angeordnet sein. Er kann jedoch alternativ oder zusätzlich auf der Aussenseite oder stirnseitig des Mantels 12 angeordnet sein. Vorzugsweise ist das Dichtelement 13 selbst haftend, insbesondere klebend, bezüglich der Abdeckung 3 ausgebildet.

Innerhalb der dichtenden Fläche des Grundkörpers 11, hier somit innerhalb des Dichtungselements 13, sind die Auflagewand 17 und die Durchdringungselemente 14 angeordnet.

Die Auflagewand 17 schliesst den Hohlraum 16 des Grundkörpers wundseitig ab. Diese Auflagewand 17 ist vorzugsweise durch eine Membran bzw. eine flexible Wand gebildet, welche luftdicht mit dem Mantel 12 des Grundkörpers 11 verbunden ist. Sie verläuft vorzugsweise fluchtend mit der unteren Stirnfläche des Mantels 12 bzw. des Dichtelements 13. Sie kann aber auch zu diesen zurückversetzt oder vorstehend angeordnet sein.

Die Auflagewand 17 ist so flexibel ausgebildet, dass sie sich an die Oberfläche der Abdeckung 3 anpasst und vorzugsweise annähernd über ihre gesamte Fläche auf der Abdeckung 3 anliegt.

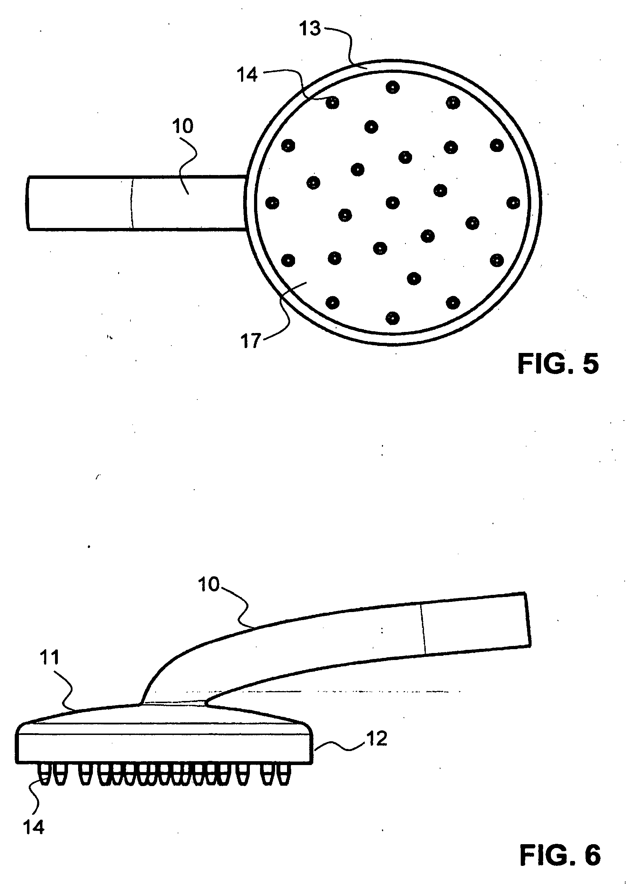

Die Auflagewand 17 weist mindestens ein Durchstossungs- bzw. Durchdringungselement 14 auf. In diesem Beispiel sind mehrere Durchdringungselemente 14 vorhanden. Diese Durchdringungselemente 14 sind vorzugsweise über die gesamte Fläche der Auflagewand 17 verteilt angeordnet, wie dies beispielsweise in den Figuren 2 und 5 erkennbar ist. Vorzugsweise sind sie gleichmässig verteilt angeordnet.

Diese Durchdringungselemente 14 überragen die untere Stirnfläche des Grundkörpers 11 und die Auflagewand 17 und stehen wundseitig nach aussen vor. Dies ist beispielsweise in Figur 6 erkennbar.

Wenn nun die erfindungsgemässe Verbindungsvorrichtung 1 auf die Abdeckung:3 aufgesetzt und leicht auf die Abdeckung 3 gepresst wird, so durchstossen bzw. durchdringen die Durchdringungselemente 14 diese Abdeckung 3 und erzeugen Durchgangsöffnungen in der Abdeckung. Dadurch werden durchgehende Kanäle zwischen Hohlraum 16 bzw. Drainageschlauch und Wundbett W geschaffen. Dies ist in Figur 1 erkennbar. Die Membran 17 schmiegt sich dabei vorzugsweise an die Oberfläche der Wundabdeckung 3 an.

Die Durchdringungselemente 14 sind vorzugsweise Stifte oder Rohre, insbesondere aus Metall oder Kunststoff. Sie weisen vorzugsweise eine kegelförmige oder pyramidenförmige Grundform auf. Sie sind vorzugsweise spitz und/oder schneidend ausgebildet.

Im hier dargestellten Beispiel weisen sie Durchgangskanäle 15 auf. Dabei können, müssen jedoch nicht alle Durchdringungselemente 14 mit Kanälen 15 versehen sein. Die Elemente 14 sind insbesondere als Kanülen ausgebildet, so dass die Verbindung zwischen Hohlraum 16 bzw. Drainageschlauch und Wundbett W durch diese Kanäle 15 erfolgt.

In Figur 1 ist die Anwendung der Vorrichtung als Wunddrainage-Saugkopf dargestellt. Der Anschlussstutzen 10 ist mit einem nicht dargestellten Saugschlauch einer Saugquelle verbunden. Die Wundflüssigkeit wird nun über die Durchgangskanäle 15 in den Saugraum bzw. Hohlraum 16 und von dort über den Anschlussstutzen 10 in den Saugschlauch abgesaugt. Die entsprechende Richtung ist mit Pfeilen dargestellt.

Die Vorrichtung gemäss Figur 1 könnte jedoch auch in umgekehrter Richtung verwendet werden; d.h. es könnte auch ein Gas oder eine Flüssigkeit über die Kanülen 15 in die Wunde W eingerührt werden.

Typische Abmessungen für eine derartige Vorrichtung sind:

| | |
|---|---|
| Durchmesser des Grundkörpers 11: 20 | bis 40 mm; |
| Höhe des Grundkörpers | circa 5 mm; |
| äusserer Durchmesser eines Durchdringungselements 14: | 1 bis 2 mm; |
| Höhe des Durchdringungselements 14: | 0.2 bis 5 mm und |
| innerer Durchmesser eines Kanals 15: | 0.5 mm bis 1 mm. |

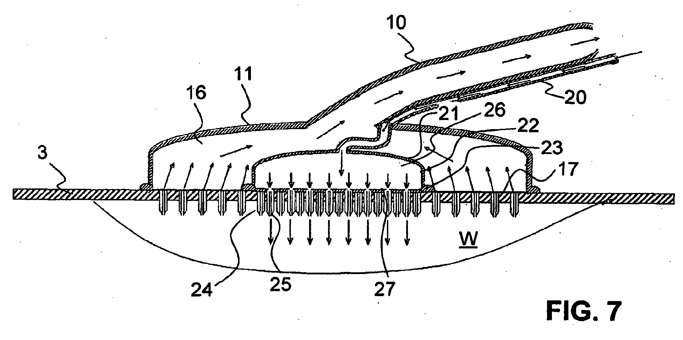

In Figur 7 ist ein Mehrzeckkopf dargestellt. Der Grundaufbau ist derselbe wie im vorherigen Beispiel, so dass gleiche Teile mit den gleichen Bezugszeichen bezeichnet sind. Im Innern des ersten Grundkörpers 11 befindet sich nun jedoch ein zweiter Grundkörper 21 mit kleinerer Grundfläche. Auch an diesem schliesst ein zweiter Anschlussstutzen 20 an, welcher ausserhalb und angrenzend zum ersten Anschlussstutzen 10 verläuft. Er könnte auch beabstandet zu diesem oder in seinem Innern verlaufen.

Der zweite Grundkörper 21 kann wie der erste Grundkörper eine runde, ovale, rechteckige, sternförmige oder andere Grundform aufweisen. Auch seine Grundfläche ist vorzugsweise um ein Vielfaches grösser als die Querschnittsfläche seines Anschlussstutzens bzw. des damit verbundenen Drainageschlauchs. Der zweite Grundkörper 21 ist hier mittig im ersten Grundkörper 11 angeordnet. Er kann jedoch auch peripher oder ausserhalb, an diesen angrenzend oder beabstandet zu ihm angeordnet sein. Er weist wiederum vorzugsweise ein seinen Mantel 22 umlaufendes Dichtelement 23, vorzugsweise einen Dichtring oder eine Dichtfläche, auf. Auch dieses Dichtelement 23 ist vorzugsweise bezüglich der Abdeckung selbsthaftend ausgebildet.

Der zweite Grundkörper 21 ist ebenfalls vorzugsweise starr bzw. formstabil gestaltet. Er ist ebenfalls nach unten zur Wunde hin offen ausgebildet und mit einer sich der Wundabdeckung 3 in der Form anpassenden Auflagewand 27, insbesondere einer elastischen Membran, verschlossen. Auch diese Auflagewand 27 ist mit Durchdringungselementen 24, insbesondere Kanülen, versehen. Vorzugsweise sind auch diese Durchdringungs-elemente 24 spitzig und/oder schneidend ausgebildet. Sie können dieselben oder grössere bzw. kleinere Abmessungen aufweisen wie die erstgenannten Durchdringungselemente 14. Insbesondere können ihre Durchgangskanäle 25 denselben oder einen grösseren bzw. kleineren inneren Durchmesser aufweisen als die Durchgangskanäle 15 der ersten Durchdringungselemente 14.

Wie in Figur 7 dargestellt ist, kann somit über die ersten Kanülen 15 die Wundflüssigkeit aus dem Wundbett abgesaugt und ohne Veränderung der Lage der erfindungsgemässen Vorrichtung über die zweiten Kanülen 25 ein Medikament, eine Flüssigkeit oder ein Gas in das Wundbett zugeführt werden. Diese Zuführung kann gleichzeitig oder zeitversetzt zur Absaugung erfolgen. Es lassen sich jedoch auch über beide Kanülen unterschiedliche Stoffe in das Wundbett zuführen. Des Weiteren kann die Zuführung über die ersten Kanülen 15 und die Absaugung über die zweiten Kanülen 25 erfolgen. Ferner ist es möglich, dass noch ein oder mehrere weitere Grundkörper mit weiteren Kanülen vorhanden sind, welche ausserhalb des ersten Grundkörpers oder in diesem angeordnet sind. In Figur 7 verlaufen die Dichtelemente 13, 23 ausserhalb der jeweiligen Grundkörper 11, 21. Sie können jedoch alternativ bzw. zusätzlich auch wiederum unterhalb oder innerhalb der Grundkörper 11,21 verlaufen.

Die erfindungsgemässe Vorrichtung ermöglicht eine einfache, schnelle und dichte Verbindung der Abdeckung mit einem Schlauch. Zudem ermöglicht sie eine möglichst gleichmässige und grossflächige Behandlung der Wunde.

### Bezugszeichenliste

- 1: Verbindungsvorrichtung

- 10: erster Anschlussstutzen
- 11: erster Grundkörper
- 12: Mantel
- 13: Dichtring
- 14: Durchdringungselement
- 15: Durchgangskanal
- 16: Hohlraum
- 17: Auflagewand

- 20: zweiter Anschlussstutzen
- 21: zweiter Grundkörper
- 22: Mantel
- 23: Dichtring
- 24: Durchdringungselement
- 25: Durchgangskanal
- 26: Hohlraum
- 27: Auflagewand

- 3: Wundabdeckung

- W: Wundbett

## Patentansprüche

1. Wundabdeckungs-Verbindungsvorrichtung (1) zur dichtenden Verbindung eines Drainageschlauchs und einer eine Wunde verschliessenden Wundabdeckung (3), wobei die Vorrichtung einen Grundkörper (11) aufweist zur dichtenden Auflage auf einer Aussenseite der Abdeckung (3), **dadurch gekennzeichnet, dass** die Vorrichtung mindestens ein Durchdringungselement (14) aufweist zur Erzeugung einer Durchgangsöffnung in der Abdeckung (3).

2. Vorrichtung nach Anspruch 1, wobei mehrere, beanstandet zueinander angeordnete Durchdringungselemente (14) vorhanden sind, welche zur Durchstossung der Abdeckung (3) an mehreren beabstandet zueinander angeordneten Stellen ausgebildet sind.

3. Vorrichtung nach einem der Ansprüche 1 oder 2, wobei die Durchdringungselemente (14) innerhalb eines dichtenden Auflagebereichs (13) des Grundkörpers (11) angeordnet sind.

4. Vorrichtung nach einem der Ansprüche 1 bis 3, wobei die Durchdringungselemente (14) spitzig und/oder schneidend ausgebildet sind.

5. Vorrichtung nach einem der Ansprüche 1 bis 4, wobei mindestens ein Teil der Durchdringungselemente (14) mit Durchgangskanälen (15) zum Absaugen der Flüssigkeit ausgebildet sind.

6. Vorrichtung nach einem der Ansprüche 1 bis 5, wobei sie ein den Grundkörper (11) umlaufendes Dichtelement (13) aufweist.

7. Vorrichtung nach Anspruch 6, wobei das Dichtungselement (13) selbst haftend ausgebildet ist.

8. Vorrichtung nach einem der Ansprüche 6 oder 7, wobei das Dichtungselement (13) auf der Aussen- und/oder der Innenseite des Grundkörpers (11) verläuft.

9. Vorrichtung nach einem der Ansprüche 1 bis 8, wobei sie einen Anschlussstutzen (10) zur Verbindung mit einer Saugquelle aufweist und wobei der Grundkörper (11) eine Grundfläche aufweist, welche um ein vielfaches grösser ist als die Querschnittsfläche des Anschlussstutzens (11).

10. Vorrichtung nach einem der Ansprüche 1 bis 9, wobei der Grundkörper (11) eine annähernd runde oder ovale Grundfläche aufweist.

11. Vorrichtung nach einem der Ansprüche 1 bis 10, wobei der Grundkörper (11) wundseitig mit einer Auflagewand (17) verschlossen ist und wobei die Durchdringungselemente (14) in dieser Auflagewand (17) angeordnet sind.

12. Vorrichtung nach Anspruch 11, wobei die Auflagewand (17) selbsttätig an die Oberfläche der Abdeckung (3) anpassbar ausgebildet ist.

13. Vorrichtung nach Anspruch 12, wobei die Auflagewand (17) elastisch ausgebildet ist.

14. Vorrichtung nach einem der Ansprüche 11 bis 13, wobei die Durchdringungselemente (14) annähernd gleichmässig verteilt über die gesamte Auflagewand (17) des Grundkörpers (11) angeordnet sind.

15. Vorrichtung nach einem der Ansprüche 1 bis 14, wobei sie mindestens einen zweiten Grundkörper (21) mit zweiten Durchdringungselementen (24) aufweist.

16. Vorrichtung nach Anspruch 15, wobei der mindestens eine zweite Grundkörper (21) im ersten Grundkörper (11) angeordnet ist.

17. Vorrichtung nach einem der Ansprüche 15 oder 16, wobei der mindestens eine zweite Grundkörper (21) ein Dichtungselement (23) zur dichtenden Auflage auf der Abdeckung (3) der Wunde aufweist.

## Claims

1. A wound cover (1) connecting device for sealingly connecting a drainage tube and a wound cover (3) closing a wound, wherein the device has a main body (11) for bearing sealingly on an outer face of the cover (3), **characterized in that** the device has at least one penetration element (14) for creating a through-opening in the cover (3).

2. The device as claimed in claim 1, wherein several penetration elements (14) are present, which are spaced apart from one another and which are designed to pierce through the cover (3) at a plurality of locations spaced apart from one another.

3. The device as claimed in either of claims 1 and 2, wherein the penetration elements (14) are arranged inside a sealing contact area (13) of the main body (11).

4. The device as claimed in one of claims 1 through 3, wherein the penetration elements (14) have a sharp-pointed and/or cutting shape.

5. The device as claimed in one of claims 1 through 4, wherein at least some of the penetration elements (14) are designed with through-channels (15) for aspirating the liquid.

6. The device as claimed in one of claims 1 through 5, wherein the device has a sealing element (13) extending around the main body (11).

7. The device as claimed in claim 6, wherein the sealing element (13) is self-adhesive.

8. The device as claimed in one of claims 6 and 7, wherein the sealing element (13) extends on the outer face and/or inner face of the main body (11).

9. The device as claimed in one of claims 1 through 8, wherein the device has an attachment piece (10) for connection to a suction source, and wherein the main body (11) has a surface area many times greater than the cross-sectional area of the attachment piece (11).

10. The device as claimed in one of claims 1 through 9, wherein the main body (11) has an approximately round or oval surface area.

11. The device as claimed in one of claims 1 through 10, wherein the main body (11), on the wound side thereof, is closed by a contact wall (17), and wherein the penetration elements (14) are arranged in this contact wall (17).

12. The device as claimed in claim 11, wherein the contact wall (17) is designed to adapt automatically to the surface of the cover (3).

13. The device as claimed in claim 12, wherein the contact wall (17) is elastic.

14. The device as claimed in one of claims 11 through 13, wherein the penetration elements (14) are distributed approximately uniformly across the entire contact wall (17) of the main body (11).

15. The device as claimed in one of claims 1 through 14, wherein the device has at least one second main body (21) with second penetration elements (24).

16. The device as claimed in claim 15, wherein the at least one second main body (21) is arranged in the first main body (11).

17. The device as claimed in one of claims 15 and 16, wherein the at least one second main body (21) has a sealing element (23) for bearing sealingly on the cover (3) of the wound.

## Revendications

1. Dispositif de liaison de pansement (1) pour la liaison étanche d'un tuyau de drainage et d'un pansement (3) fermant une plaie, le dispositif présentant un corps de base (11) pour l'application étanche sur un côté extérieur du pansement (3), **caractérisé en ce que** le dispositif présente au moins un élément de perçage (14) pour produire une ouverture de passage dans le pansement (3).

2. Dispositif selon la revendication 1, dans lequel plusieurs éléments de perçage (14) disposés à distance les uns des autres sont prévus, lesquels sont réalisés pour traverser le pansement (3) au niveau de plusieurs endroits espacés les uns des autres.

3. Dispositif selon l'une quelconque des revendications 1 ou 2, dans lequel les éléments de perçage (14) sont disposés à l'intérieur d'une région d'application étanche (13) du corps de base (11).

4. Dispositif selon l'une quelconque des revendications 1 à 3, dans lequel les éléments de perçage (14) sont réalisés sous forme pointue et/ou coupante.

5. Dispositif selon l'une quelconque des revendications 1 à 4, dans lequel au moins une partie des éléments de perçage (14) est réalisée avec des canaux de passage (15) pour aspirer le liquide.

6. Dispositif selon l'une quelconque des revendications 1 à 5, lequel présente un élément d'étanchéité (13) entourant le corps de base (11).

7. Dispositif selon la revendication 6, dans lequel l'élément d'étanchéité (13) est réalisé sous forme autocollante.

8. Dispositif selon l'une quelconque des revendications 6 ou 7, dans lequel l'élément d'étanchéité (13) s'étend sur le côté extérieur et/ou le côté intérieur du corps de base (11).

9. Dispositif selon l'une quelconque des revendications 1 à 8, lequel présente une tubulure de raccordement (10) pour la liaison à une source d'aspiration et dans lequel le corps de base (11) présente une surface de base qui est plus grande d'un multiple que la surface en section transversale de la tubulure de raccordement (11).

10. Dispositif selon l'une quelconque des revendications 1 à 9, dans lequel le corps de base (11) présente une surface de base approximativement ronde ou ovale.

11. Dispositif selon l'une quelconque des revendications 1 à 10, dans lequel le corps de base (11) est fermé du côté de la plaie par une paroi d'application (17) et dans lequel les éléments de perçage (14) sont disposés dans cette paroi d'application (17).

12. Dispositif selon la revendication 11, dans lequel la paroi d'application (17) est réalisée de manière à pouvoir s'adapter automatiquement à la surface du pansement (3).

13. Dispositif selon la revendication 12, dans lequel la paroi d'application (17) est réalisée sous forme élastique.

14. Dispositif selon l'une quelconque des revendications 11 à 13, dans lequel les éléments de perçage (14) sont répartis approximativement uniformément sur toute la paroi d'application (17) du corps de base (11).

15. Dispositif selon l'une quelconque des revendications 1 à 14, lequel présente au moins un deuxième corps de base (21) avec des deuxièmes éléments de perçage (24).

16. Dispositif selon la revendication 15, dans lequel l'au moins un deuxième corps de base (21) est disposé dans le premier corps de base (11).

17. Dispositif selon l'une quelconque des revendications 15 ou 16, dans lequel l'au moins un deuxième corps de base (21) présente un élément d'étanchéité (23) pour l'application étanche sur le pansement (3) de la plaie.
